# EUROPEAN PATENT APPLICATION

(11) **EP 2 548 606 A1**
(43) Date of publication of application: **23.01.2013**
(21) Application number: 11174690.5
(22) Date of filing: 20.07.2011
(51) Int. Cl.: A61M 25/10, A61B 5/00

(54) **Catheter for creating a spatially separated vessel section**

(71) Applicant: Netzer, Florian, 80802 München (DE)
(72) Inventor: Netzer, Florian, 80802 München (DE)
(74) Representative: Maiwald Patentanwalts GmbH

(57) **Abstract**

The present application relates to a catherer wherein the catherer provides for two inflatable baloon devices which are configured to perform a relative movement with respect to each other. By uncovering an opening upon distancing the two inflated baloon devices, a confined and spatially separated vessel section is created in which optical energy may be emitted to treat the vessel.

## Description

### FIELD OF THE INVENTION

The present invention relates to catheter technology. In particular, the present invention relates to a catheter for creating a spatially separated vessel section and a method for creating a spatially separated vessel section.

### BACKGROUND OF THE INVENTION

In medicine, a catheter is a tube that can be inserted into a body cavity, duct or vessel. Catheters thereby allow drainage, administration of fluids or gases, or access by surgical instruments. In most uses, a catheter is a thin, flexible tube and may be called a soft catheter. In some other uses, it is a larger, solid catheter which may be named a hard catheter. Moreover, a balloon catheter is known in the art as a type of soft catheter with a single **i**nflatable balloon at its tip which is used during a catheterization procedure to enlarge a narrow opening or passage within a body. The deflated balloon catheter is positioned, then inflated to perform the necessary procedure, and deflated again in order to be removed. By means of mechanical pressure, narrow passages within a vessel may be spatially broadened due to the applied force of the inflated balloon.

In case a balloon catheter is inserted into a blood vessel and the user desires to insert specific surgical instruments via the catheter, the application of said surgical instruments will always take place within a vessel which is full of blood. However, certain surgical instruments or certain applications may suffer from disadvantages caused by the presence of the blood at the desired position.

### SUMMARY OF THE INVENTION

It may be seen as an object of the present invention to provide for an improved catheter technology.

There may be a need to provide for a spatially separated vessel section within, for example, a blood vessel in order to allow specific vessel treatment in said created spatially separated vessel section.

The object of the present invention is solved by the subject-matter of the independent claims. Further advantages and embodiments are incorporated in the dependent claims.

It should be noted that the invention in the following will be described with regard to a catheter and thus will be described with respect to apparatus type claims. However, features and advantages described in context of the catheter do similarly apply and relate to the below presented method for creating a spatially separated vessel section, and vice versa.

According to an exemplary embodiment of the invention, a catheter for creating a spatially separated vessel section is presented. The catheter comprises a tube for inserting into a blood vessel, a first balloon device and a second balloon device. Furthermore, at least one of the first balloon device and the second balloon device is movably arranged at the tube. Moreover, the movable arrangement of the at least one of the first balloon device and the second balloon device enables for a change of distance between the first balloon device and the second balloon device. Consequently, the desired separated vessel section can be established i.e. the spatial separation can be completed within the vessel.

In the context of the present invention the distance between the two balloon devices may be caused by a distancing mechanism. As will be described below several possibilities may be used to embody said distancing mechanism. For example a tubular system comprising three coaxially tubes may be used as defined below.

The tube of the catheter may extend between the two balloon devices. Furthermore the catheter may be embodies as a tubular system comprising three tubes, wherein the first tube is inside of the second and the third tube, whereas the second tube is inside the third tube. Thus, the first tube may be seen as the inner tube, the second tube may be seen as the medium tube and the third tube may be seen as the outer tube.

In such a tubular catheter system the first tube is embodied as a laser catheter. The second tube may comprise the first balloon device and the third tube may comprise the third balloon device. The three tubes may be embodied coaxial such that a relative translation of the tubes with respect to each other is possible by means of e.g. shifting at least one of the tubes. Consequently, the three tubes have three different diameters. If desired, the respective balloon devices may be positioned at the end of the respective tube. By means of translating the third tube relative to the second tube the two balloon devices may be brought in the desired distanced position as described above and below hereinafter. In case of such a tubular system, the opening of the catheter used to release a medium into the treated blood vessel may be comprised by the second tube. The second tube may be shifted or displaced within the third, outer tube such that the opening is uncovered upon shifting the second tube relative to the third, outer tube. If desired, such a tubular catheter system may be configured to simultaneously shift the first and the second tube relative to and within the third tube.

Furthermore, in this and in every other embodiment of the invention the tube may be of transparent material with regard to the wavelength of light which is used. In other words the tube and the wavelength of light which is used may be adapted accordingly according to the present invention.

The spatially separated vessel section may be seen as a vessel section that is spatially confined by the distanced first and second balloon devices and the surrounding walls of the vessel.

In other words, the first and the second balloon device may be attached to the tube of the catheter in such a way that they may be e.g. pulled away from each other into a distanced position. The first balloon device and the second balloon device may e.g. also distanced from another by using the catheter when they are already in an inflated state. If desired, the presented catheter may be inserted into, for example, a blood vessel when the first and second balloon devices are not inflated. After having directed the catheter to its desired location, the first and second balloon devices may be inflated by means of e.g. using an additionally provided medium supply line. Therein, the term "medium" shall be understood in the context of the present invention as gas or liquid or steam of an element or of a composition. This detail will be described hereinafter in more detail.

After the first and second balloon devices are inflated, a distancing mechanism provided by the catheter may be used in order to change especially enlarge the distance between the inflated first and second balloon devices. Such a distancing mechanism may be provided e.g. by translating one of the tubes in a tubular system, as already described above. By using such a distancing mechanism, a spatially separated vessel section is created within the blood vessel by the presented catheter. Such a spatially separated vessel section may then be present as spatially confined by the walls of the blood vessel and the inflated first and second balloon devices. Therefore, a spatial isolation of the vessel section from the remaining vessel parts can be realized by means of distancing the two balloon devices. Said two balloon devices may be filled with a medium like a gas or a fluid through the catheter.

By providing the ability of the presented catheter to allow for a relative movement of the first and second balloon devices with respect to each other, a blood-free zone, the spatially separated vessel section, can be generated by the presented catheter. In sequential steps, the first and second balloon devices may firstly be inflated and afterwards may secondly be distanced from each other when being positioned in the desired vessel.

In the context of the present invention, the term "blood vessel" exemplarily describes a desired destination of the catheter. If the user desires to position the catheter in an organ, artery, vein, or any other cavity comprised by a human body, he may also use the catheter and the comprised tube according to the present invention.

Especially in the case of two or more tissue layers being positioned adjacently close together, the present invention provides for a simple possibility to create an inflated tissue free section of the body. For example a tissue free vessel section may be created between the lungs and the diaphragm by means of the present invention.

Especially in case of malfunctions of venous valves, the presented catheter may provide for special advantages. For example, in case of a treatment of the region around venous valves with optical energy provided by e.g. an optical fiber which guides laser light to such venous valves and/or the walls of the vein, a spatially separated vein section is highly desirable. This desire is met by the presented catheter as the first balloon device and the second balloon device are attached to the tube in such way that the attachment allows for a change of distance between the first balloon device and the second balloon device in an inflated stated when they are positioned in the vein.

According to an exemplary embodiment of the invention, the tube comprises a longitudinal axis and at least one of the first balloon device and the second balloon device is movable along the longitudinal axis of the tube.

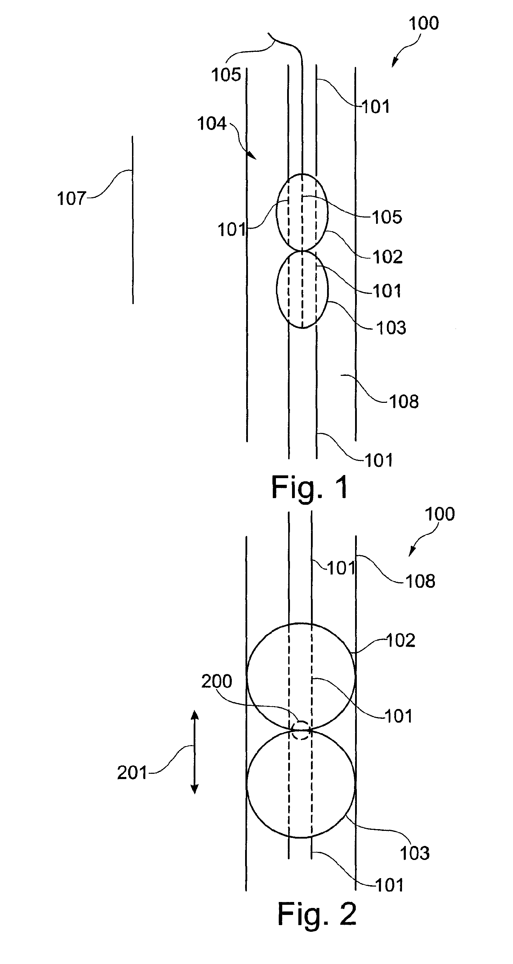

Such a longitudinal axis may be gathered from the following Fig. 1 and the herein presented description of Fig. 1. In other words, the presented embodiment allows for a translational movement of at least one of the first and second balloon device, if the catheter is placed within a blood vessel. If desired, one or also both balloon devices may be inflated before being translated along the longitudinal axis of the tube, such that the spatial confinements, i.e. the walls of the balloon device push out, displace, or suppress blood within the blood vessel when the balloon device is translated along the tube. The previously described procedure allows for establishing a blood-free vessel section or an inflated tissue free section of the body (Hallo Herr Dr. Netzer: bitte erläutern sie diese section in wenigen Sätzen.) which is spatially separated as two balloon devices may be inflated to such an extent that they spatially adjoin or border directly to the inner surfaces of the blood vessel wall. In other words, when being inflated and distanced from each other, the two balloon devices prevent a blood flow into the vessel section that is located between the two distanced balloon devices.

The presented catheter may allow to separately and independently moving the first and the second balloon device with the moving mechanism i.e. the distancing mechanism. But also the embodiments are comprised by the present invention in which only one of the balloon devices is movable and the respective other one is fixed in its position at the tube.

According to an exemplary embodiment of the present invention, the catheter is for treating a blood vessel with optical energy, and the tube is adapted to receive an optical fiber. Furthermore, the tube comprises an opening for releasing optical energy of the received optical fiber from inside the tube towards the blood vessel in which the tube is inserted.

Especially in case of malfunctions of venous valves, such an optical catheter enables the user to treat the vessel in the spatially separated vessel section by means of optical energy, which is especially advantageous. If desired, the catheter may provide for a radial emission of the electromagnetic energy in order to treat the spatially separated vessel section in a uniform and isotropic way. Therefore, the optical fiber may be comprised by the catheter. Furthermore, the catheter may allow for a rotation of the optical fiber within the catheter such that by rotating the optical fiber, a circular emission of the laser light coupled to the fiber towards the vessel wall is provided.

The person skilled in the art directly and unambiguously derives from the above described details that several different optical fibers and several different light sources may be combined in combination with the presented catheter.

According to an exemplary embodiment, the first and second balloon devices are movably arranged at the tube in such a way that upon an increase of said distance between the first and the second balloon devices, the first and second balloon devices are positioned at opposing positions of the opening for releasing optical energy.

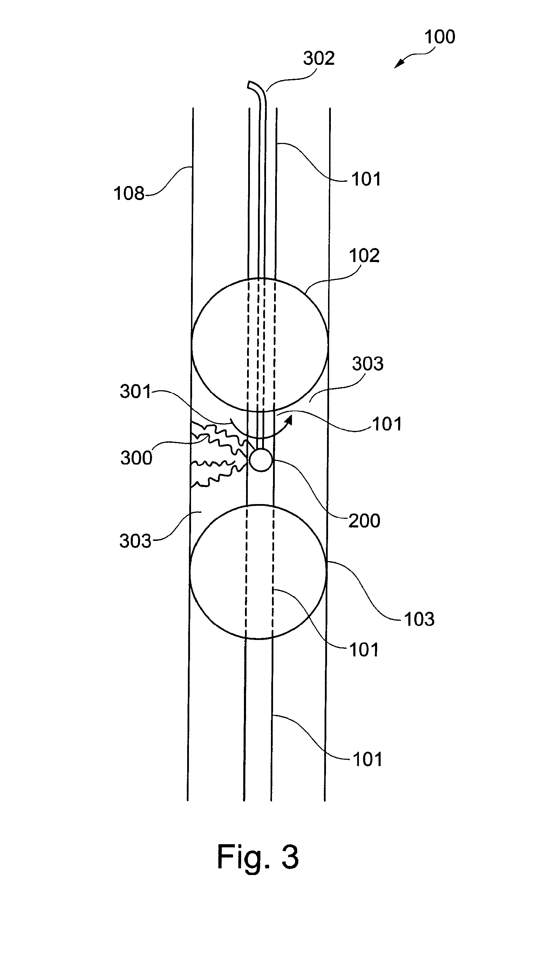

As can be, for example, gathered from Fig. 3, the catheter allows for a translation of the two balloon devices along the longitudinal axis upon which translation the opening 200 is uncovered. In other words, the catheter allows for an uncovering of the opening for releasing optical energy by means of causing or forcing at least one of the first and second balloon devices away from its adjacent position from the respective other balloon device. If desired, also both balloon devices may be forced to translate in opposite directions on order to uncover the opening and to simultaneously create the above and below described spatially separated vessel section.

According to an exemplary embodiment, at least one medium supply line like a gas and / or fluid supply line is provided by the catheter. The gas supply line may be directed to the first balloon device and the second balloon device.

For example, the catheter may comprise one medium supply line for both balloon devices. In another embodiment two separate supply lines for the two balloon devices may be comprises. In any case the catheter may be configured in combination with the supply line or the two supply lines such that a separate inflation and/or deflation of the balloon devices is possible.

If desired a corresponding gas, steam and/or fluid storage device may be comprised in this and every other embodiment of the present invention.

In order to inflate the first and second balloon device after they were inserted into the desired blood vessel, a directing of gas along the catheter to the balloon devices is possible and provided by the present invention.

According to another exemplary embodiment of the invention, the first balloon device and the second balloon device are inflatable by supplying gas to the first and the second balloon device via the gas supply line.

According to another exemplary embodiment of the invention, the catheter comprises an optical fiber for guiding optical energy of a light source through the catheter and to release the optical energy in the separated vessel section, a detector for detecting the optical energy, which is released in the separated vessel section. the catheter further comprises a control unit for receiving a detector signal generated by the detector, wherein the control unit is configured to control a light source based on the received detector signal.

If desired, the catheter may also comprise a light source like e.g. a laser. The laser may be single wavelength laser or may also be embodied as broadband source. The light source may also be embodied as a pulsed laser. The light source may also be embodied as a tunable light source, being tunable with respect to wavelength, polarization, power, pulse form and / or pulse duration.

Said control unit may be connected by wire or wireless with the detector and the light source. In other words a catheter for laser treatment of a blood vessel is presented which comprises a feedback loop or feedback line with regard to the released optical energy. Therein the term "released optical energy" shall be understood as the electromagnetic energy which is emitted in the blood vessel. Therefore, the user is provided with a control of the energy, to which the blood vessel is exposed. This may ensure medical and / or safety requirements.

Especially when releasing a medium like a gas or a liquid into the blood vessel via the catheter, the detector provides for a control signal. The type and the amount of the medium, which is released inside the blood vessel, may change the amount of optical energy that is absorbed by the blood vessel as the optical properties of the propagation path of the light are controllably changed. Hence based on the signal generated by the detector the inflow and outflow of the medium may be controlled e.g. by means of the control unit. The detector may be configured to detect the amount of optical energy which is emitted into the blood vessel.

If desired, a predetermined and desired value of the optical energy that is to be released may be defined such that a regulation of the optical power of the light source is provided to converge to the desired value based on the signal generated by the detector of the catheter. The control unit may be configured to perform such a regulation. For example a desired value may be 75 kJ/cm² per wall of vene. However, the scope of the present invention is not left, if other values are chosen as this value is only exemplary.

However, if desired the detector of the catheter may also be configured to detect other physical parameters like wavelength, polarization, power, pulse form and / or pulse duration may be controlled by means of the present invention by inserting a medium like gas or a liquid. Therefore, a control device may be comprised by the catheter of the present invention which is configured to control said physical parameters like e.g. the absorption rate at the vessel or tissue by means of for example a detector.

In other words the present invention allows for the generation of a separated vessel section in which the optical properties of the propagation path of the light are controllably changed in order to provide for a improved vessel or tissue treatment.

According to another exemplary embodiment of the present invention the catheter comprises a steam supply line. The steam supply line may be connected with the opening of the tube of the catheter such that the catheter is configured to apply steam to the blood vessel. Steam may be used in order to shrink the diameter of the treated blood vessel according to the present invention. Furthermore, if desired, the steam may be applied such that the blood vessel like e.g. a vene is completely closed by means of steam application through the catheter.

The steam supply line may be arranged at the catheter like e.g. the fiber 105 shown in fig. 1 and as is described above and hereinafter in detail. If desired, the catheter of the present invention may comprise a steam generating device and / or a steam storage device. However, also other arrangements of such a steam supply line are possible.

According to another exemplary embodiment of the present invention, the first balloon device and the second balloon device are movable from a minimal distant position to a maximal distant position, and the first and the second balloon devices are positioned adjacently to each other at the tube when they are in the minimal distant position.

As may be seen in Fig. 1, a minimal distant position provides for direct contact between the first and the second balloon device. In a maximal distant position, which may be seen in Fig. 3, the balloon devices are positioned at opposing positions of the opening. Thereby, the spatially separated vessel section is created and an uncovering of the opening for emitting for example optical energy or for example ultrasound energy is provided. Thereby space for other surgical instruments as e.g. endoscopes is advantageously generated.

According to another exemplary embodiment of the invention, the catheter is adapted to allow for sequentially inflating and distancing of the first and second balloon devices in such a way that firstly the first and the second balloon devices are inflatable in an adjacent position, and is further adapted in such a way that secondly the distance between the first and second balloon device is adjustable in an inflated state.

This process may be gathered from the below described Figs. 1 to 3. However, the present invention may also allow to firstly translate the balloon devices to a desired position, afterwards inflate the balloon devices, and if desired an additional translation or position of the balloon devices may formed by means of the provided catheter.

According to another exemplary embodiment, the catheter further provides for a second opening for releasing a medium like e.g. a gas or a liquid into a vessel section that is spatially confined by the distanced first and second balloon devices.

By means of providing for a first and second opening, optical energy may be released simultaneously to the release of, for example, an inert gas in order to improve the vessel treatment or tissue treatment by optical energy. Inert gas and / or fluid may be used in this and also in any other exemplary embodiments of the invention in order to reduce chemical reactions taking place during application with the catheter according to the present invention. Furthermore physical parameters like optical absorption may be controlled by means of the present invention by inserting a medium like gas or a liquid. Therefore, a control device may be comprised by the catheter of the present invention which is configured to control said physical parameters like e.g. the absorption rate at the vessel or tissue by means of for example a detector. For example, the optical treatment of the vessel in the spatially separated vessel section may be improved as inert gas may be directed through the catheter into such a spatially separated vessel section.

According to another exemplary embodiment of the invention, a method for creating a spatially separated vessel section is presented. The method comprises the steps of providing for a catheter comprising a tube, a first balloon device and a second balloon device. Therein, the first balloon device and the second balloon device are arranged at the tube of the catheter in a non-inflated state and adjacently positioned. In a further step, the presented method provides for inflating the first balloon device and inflating the second balloon device, and increasing a distance between the first balloon device and a second balloon device.

If desired, the provided steps may also be formed in another order such that firstly an increasing of a distance is performed and subsequently, an inflating of the balloon devices is performed. If desired, an additional increasing of the distance may be caused which may be seen as distancing the first and the second balloon devices.

According to another exemplary embodiment of the invention, the method comprises the step of sealing the vessel with regard to blood flow by means of inflating the first and the second balloon devices.

According to another exemplary embodiment of the invention, the method comprises the step of creating a spatially separated vessel section upon increasing the distance between the first and the second balloon device, wherein a vessel section is spatially confined by the first balloon device and the second balloon device.

This process may be gathered additionally from Figs. 1 to 3 in which a spatially separated vessel section is translated by means of inflating and translating the first and second balloon devices. However, it is also possible that only one of the balloon devices is translated, whereas the other one stays fixed in its position.

According to another exemplary embodiment of the invention, the presented method is for treating a blood vessel with optical energy and comprises the step of emitting optical energy into the spatially separated vessel section through a first opening of the tube of the catheter.

According to another exemplary embodiment of the invention, the method provides for emitting a medium like a gas or a fluid in the separated vessel section through the tube.

This method step may be performed by means of a second opening provided by the tube. However, the gas may also be inserted into the separated vessel section through the first opening through which also the optical energy is emitted. If desired, inert gas or a fluid may used in order to improve the application of optical energy to the vessel section.

In this and every other embodiment of the invention the insertion of a therapeutical gas or fluid may be used. The application of such a medium e.g. in between the two distanced inflated balloon devices may improve the surgical treatment of the patient.

According to another exemplary embodiment of the invention, the step of increasing the distance between the first balloon device and the second balloon device is carried out by translating at least one of the first balloon device and the second balloon device along a longitudinal axis of the tube of the catheter in an inflated state.

According to another exemplary embodiment of the invention, the method comprises the step of uncovering a first opening of the tube for releasing optical energy between the distanced first and second balloon device upon increasing the distance of the first and second balloon device.

In order to increase the distance of the first and second balloon device, a push mechanism or also a pull mechanism may be used in order to change the absolute position of at least one of the first and second balloon device. Therein mechanical mechanisms, electrical mechanisms, electronical mechanisms and any combination thereof may be provided. Such a mechanism may also be used to translate both of the balloon devices. If desired, also a rotational component of the movement of the movable balloon device may be caused by the presented method.

It can be seen as a gist of the invention to generate or establish a spatially separated vessel section by means of two inflatable balloon devices that are caused to perform a relative movement to each other in order to uncover an opening. The movement is caused by means of the catheter. The opening is comprised by the tube of the catheter and allows for emitting for example optical energy into the spatially separated vessel section. In other words, the vessel section is spatially confined by the walls of the vessel and by the distanced first balloon device and second balloon device which may be inflated or non-inflated.

These and other aspects of the invention will become apparent from and elucidated with reference to the embodiments described hereinafter.

### BRIEF DESCRIPTION OF THE DRAWINGS

Exemplary embodiments of the invention will be described in the following drawings.
Fig. 1 schematically shows a catheter according to an exemplary embodiment of the invention.
Fig. 2 schematically shows a catheter according to an exemplary embodiment of the invention.
Fig. 3 schematically shows a catheter according to an exemplary embodiment of the invention.
Figs. 4 and 5 schematically show a respective flow diagram of a method according to respective exemplary embodiments of the invention.

### DETAILED DESCRIPTION OF EMBODIMENTS

Fig. 1 shows a catheter 100 for creating a spatially separated vessel section of the shown vessel 108. The catheter comprises a tube 101 which is adapted to be inserted into the vessel. The catheter comprises a first balloon device 102 and second balloon device 103. The first balloon device and the second balloon device are movable arranged at the tube 101. The movable arrangement 104 of the at least one of the first balloon device and the second balloon device enables for a change of distance between the first balloon device and the second balloon device. Such a change of distance can be gathered from the following description, especially from the description regarding Fig. 3. The first balloon device and the second balloon device are movable along a longitudinal axis of the tube, which is symbolically shown by line 107 in Fig. 1.

In addition, a gas supply line 105 is schematically shown in Fig. 1. This may also be embodied as a liquid supply line. If desired a corresponding tank may be present. The gas supply line 105 is directed towards the first balloon device 102 and to the second balloon device 103. As the tube 101 as well as the gas supply line 105 is partially covered by the first balloon device and second balloon device, the respective lines are depicted in dashed lines in the region of the balloon devices.

The gas supply line supplies gas, for example inert gas, to the balloon devices in order to inflate both balloon devices. Such an inflating process may be seen in the following Fig. 2.

Fig. 2 schematically shows that the balloon devices 102 and 103 are inflated to such an amount that the lateral extent of the balloon devices leads to a direct contact with the walls of the vessel 108. In other words, a sealing of the vessel is provided by means of inflating the first and second balloon devices. Said two balloon devices may be filled with gas of fluid through the catheter.

As can be gathered from Fig. 2, an opening 200 is provided by tube 101. Arrow 201 depicts the movement that is caused to the first and second balloon devices 102 and 103 by means of a moving mechanism which is not shown in this figure. Such a moving mechanism or translating mechanism may be embodied in various ways.

However, such a mechanism allows for change of distance between the first balloon device and the second balloon device after having sealed the vessel 108.

As can be seen in Fig. 3, the catheter 100 of Fig. 1 and Fig. 2 is depicted in a different state. Tube 101 is still inserted into the vessel 108 and an optical fiber 302 is guided through tube 101 and passes the first balloon device 102 by means of being guided through the tube. Opening 200 allows for emitting optical energy 300 into the spatially separated vessel section which is depicted with reference sign 303. Arrow 301 symbolically depicts that a rotation of the optical fiber 302 can be performed if desired. Therefore, a radial emission of optical energy may be allowed. The first balloon device 102 and the second balloon device 103 are shown in a distanced state in which they confine the spatially separated vessel section 303. Therefore, a blood flow from the upper vessel section and the lower vessel section is prevented. Thus, a protected treatment of the vessel in the vessel section 303 can be performed by, for example, applying optical energy.

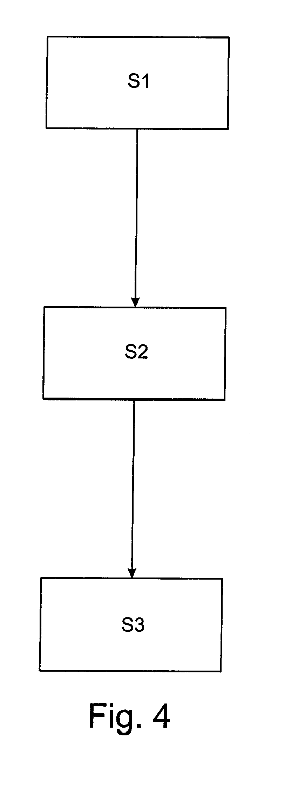

Fig. 4 shows a flow diagram of a method for creating a spatially separated vessel section. The method comprises the steps of providing for a catheter comprising a tube, a first balloon device and a second balloon device, which is depicted as step S1. Thereby, the first balloon device and the second balloon device are arranged at the tube of the catheter in a non-inflated state and adjacently positioned. In a further step S2, inflating the first balloon device and inflating the second balloon device is performed in the presented method. Furthermore, increasing a distance between the first balloon device and a second balloon device is performed during step S3. If desired, the step S3 may be carried out by translating at least one of the first balloon device and the second balloon device along the longitudinal axis of the tube of the catheter in an inflated state, as may be gathered from the combination of previously described Figs. 2 and 3. However, the individual steps of the method may also be performed in another order compared to the one which is presented in Fig. 4.

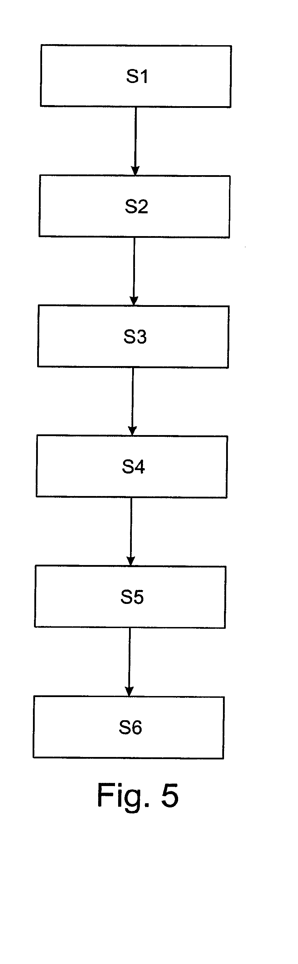

Fig. 5 shows another flow diagram of a method for creating a spatially separated vessel and for treating a blood vessel with optical energy. Steps S1, S2, and S3 arc analogously performed as they have been described with regard to Fig. 4. Therefore, for S1 to S3, reference is made to Fig. 4. In order to allow for an improved optical treatment of the vessel, step S4 is additionally performed. Creating a spatially separated vessel section upon increasing the distance between the first and the second balloon device may be seen as step S4. Consequently, the vessel section is spatially confined by the first balloon device and the second balloon device.

By emitting optical energy into the spatially separated vessel section through a first opening of the tube of the catheter in step S5, malfunctions of, for example, venous valves may be decreased by the presented method.

## Claims

1. Catheter (100) for creating a spatially separated vessel section (303), the catheter comprising:
a tube (101) for inserting into a vessel,
a first balloon device (102),
a second balloon device (103),
wherein at least one of the first balloon device and the second balloon device is movably arranged at the tube, and
wherein the movable arrangement (104) of the at least one of the first balloon device and the second balloon device enables for a change of distance between the first balloon device and the second balloon device.

2. Catheter according to claim 1,
wherein the at least one of the first balloon device and the second balloon device is movable along a longitudinal axis (107) of the tube.

3. Catheter according to one of the claims 1 or 2,
wherein the catheter is for treating a blood vessel with optical energy, wherein the tube is adapted to receive an optical fiber (302), and
wherein the tube comprises an opening (200) for releasing optical energy of the received optical fiber from inside the tube towards the blood vessel in which the tube is inserted.

4. Catheter according to one of the claims 1 to 3,
wherein the first and second balloon devices are movably arranged at the tube in such a way that upon an increase of said distance between the first and second balloon devices the first and second balloon devices are positioned at opposing positions of the opening.

5. Catheter according to one of the preceding claims, the catheter further comprising
at least one medium supply line,
wherein the at least one medium supply line is directed to the first balloon device and the second balloon device.

6. Catheter according to claim 5,
wherein the first balloon device and the second balloon device are inflatable by supplying a medium to the first and the second balloon device via the medium supply line.

7. Catheter according to one of the preceding claims, the catheter further comprising
an optical fiber for guiding optical energy of a light source through the catheter and to release the optical energy in the separated vessel section,
a detector for detecting the optical energy, which is released in the separated vessel section,
a control unit for receiving a detector signal generated by the detector, and wherein the control unit is configured to control a light source based on the received detector signal.

8. Catheter according to one of the preceding claims,
wherein the first balloon device and the second balloon device are movable from a minimal distant position to a maximal distant position, and
wherein the first and the second balloon devices are positioned adjacently to each other at the tube when they are in the minimal distant position.

9. Catheter according to one of the preceding claims,
wherein the catheter is adapted to allow for sequentially inflating and distancing of the first and second balloon devices in such a way that firstly the first and the second balloon devices are inflatable in an adjacent position and in such a way that secondly the distance between the first and second balloon device is adjustable in an inflated state.

10. Catheter according to one of the preceding claims, the tube further comprising
a second opening for releasing a medium into the spatially separated vessel section which is spatially confined by the distanced first and second balloon devices.

11. Method for creating a spatially separated vessel section, the method comprising the steps of
providing for a catheter comprising a tube, a first balloon device and a second balloon device (S1),
wherein the first balloon device and the second balloon device are arranged at the tube of the catheter in a non inflated state and adjacently positioned,
inflating the first balloon device and inflating the second balloon device (S2), and
increasing a distance between the first balloon device and a second balloon device (S3).

12. Method according to claim 11, further comprising the step of creating a spatially separated vessel section (S4) upon increasing the distance between the first and the second balloon device, and
wherein a vessel section is spatially confined by the first balloon device and the second balloon device.

13. Method according to one of claim 11 or 12,
wherein the method is for treating a blood vessel with optical energy,
the method further comprising the step of
emitting optical energy into the spatially separated vessel section through a first opening of the tube of the catheter (S5).

14. Method according to one of claims 11 to 13, further comprising the step of
emitting a medium in the separated vessel section through the tube (S6).

15. Method according to one of claims 11 to 14, further comprising the step of
uncovering a first opening of the tube for releasing optical energy between the distanced first and second balloon device upon increasing the distance of the first and second balloon device.
